# EUROPEAN PATENT SPECIFICATION

(11) **EP 2 493 837 B1**
(45) Date of publication and mention of the grant of the patent: **23.04.2014**
(21) Application number: 09748840.7
(22) Date of filing: 29.10.2009
(51) Int. Cl.: C07C 29/48

(54) **HYDROCARBON SELECTIVE OXIDATION WITH HETEROGENOUS GOLD CATALYSTS**
KOHLENWASSERSTOFFSELEKTIVE OXIDATION MIT HETEROGENEN GOLD KATALYSATOREN
OXIDATION SELECTIVE D'HYDROCARBURES AVEC DES CATALYSEURS HÉTÉROGÈNES À BASE D'OR

(43) Date of publication of application: 05.09.2012
(73) Proprietor: University College Cardiff Consultants Ltd., Cardiff CF24 0DE (GB)
(72) Inventor: LOPEZ-SANCHEZ, Jose, Antonio, Cardiff CF10 3AT (GB); DIMITRATOS, Nikolaos, Cardiff CF10 3AT (GB); JENKINS, Robert, Leyshon, Cardiff CF10 3AT (GB); CARLEY, Albert, Frederick, Cardiff CF10 3AT (GB); WILLOCK, David, James, Cardiff CF10 3AT (GB); TAYLOR, Stuart, Hamilton, Cardiff CF10 3AT (GB); HUTCHINGS, Graham, John, Cardiff CF10 3AT (GB); RAHIM, Mohd, Hasbi, Cardiff CF10 3AT (GB)
(74) Representative: Beck Greener
(86) International application number: PCT/GB2009/051461
(87) International publication number: WO 2011/051642

(56) References cited:
- GB-A- 1 041 046
- US-A1- 2006 293 175
- KALVACHEV Y A ET AL: "Vapor-Phase Selective Oxidation of Aliphatic Hydrocarbons over Gold Deposited on Mesoporous Titanium Silicates in the Co-Presence of Oxygen and Hydrogen" JOURNAL OF CATALYSIS, ACADEMIC PRESS, DULUTH, MN, US LNKD- DOI:10.1006/JCAT.1999.2540, vol. 186, no. 1, 15 August 1999 (1999-08-15), pages 228-233, XP004443112 ISSN: 0021-9517
- YI-JUN XU ET AL: "Selective conversion of cyclohexane to cyclohexanol and cyclohexanone using a gold catalyst under mild conditions" CATALYSIS LETTERS, KLUWER ACADEMIC PUBLISHERS-PLENUM PUBLISHERS, NE, vol. 101, no. 3-4, 1 June 2005 (2005-06-01), pages 175-179, XP019275159 ISSN: 1572-879X
- GASIOR M ET AL: "Oxidation of CO and C3 hydrocarbons on gold dispersed on oxide supports" CATALYSIS TODAY, ELSEVIER, NL LNKD- DOI:10.1016/J.CATTOD.2004.03.021, vol. 91-92, 15 July 2004 (2004-07-15), pages 131-135, XP002336267 ISSN: 0920-5861
- LIGNIER ET AL: "Insight into the free-radical chain mechanism of gold-catalyzed hydrocarbon oxidation reactions in the liquid phase" CATALYSIS TODAY, ELSEVIER, NL LNKD- DOI:10.1016/J.CATTOD.2007.02.006, vol. 122, no. 3-4, 10 May 2007 (2007-05-10), pages 284-291, XP022068745 ISSN: 0920-5861
- M.D.HUGHES ET AL: "Tunable gold catalysts for selective hydroncarbon oxidation under mild conditions" NATURE, vol. 437, 2005, pages 1132-1135, XP009136486
- ZHAO R ET AL: "A HIGHLY EFFICIENT OXIDATION OF CYCLOHEXANE OVER AU/ZSM-5 MOLECULAR SIEVE CATALYST WITH OXYGEN AS OXIDANT" JOURNAL OF THE CHEMICAL SOCIETY, CHEMICAL COMMUNICATIONS, CHEMICAL SOCIETY. LETCHWORTH, GB, vol. 7, 1 January 2004 (2004-01-01), page 904/905, XP009071039 ISSN: 0022-4936

## Description

This invention relates to processes and catalysts that convert C₁-C₈ hydrocarbons to a corresponding alcohol, such as methane to methanol, using a supported heterogeneous gold catalyst

Activation and oxidation of lower alkanes (C₁-C₃) into useful oxygenates has long been an attractive and challenging research area. The reason for this intense interest is due to the fact that the lower alkanes (C₁-C₃) are the main constituents of natural gas, which is rather inexpensive and in high abundance. Therefore, it is desirable to transform the inexpensive and abundant lower alkanes to useful chemicals. The activation of lower alkanes usually requires severe conditions using heterogeneous catalysts (high temperature > 500 °C and potentially pressure) because of their chemical inertness. However, under these reaction conditions the valuable oxygenated products are not stable and the significant formation of carbon oxides (CO and CO₂) is usually observed at relevant conversions.

Therefore, it is considered desirable to work at milder conditions, where the COₓ formation will not prevail and the stability of the oxygenated products formed will be greater. The alternative solution for the activation of methane at lower reaction temperatures is to work in the liquid phase instead of the gas phase. Recently, several groups have tried the oxidation of methane in the liquid phase using a pressurized reactor and temperature below 200 °C. However, many of these groups have used strong acid media such as sulfuric acid; therefore the reaction conditions are corrosive and toxic, creating a large amount of waste. Specifically, B. Michalkiewicz et al., J. Catal. 215 (2003) 14, have reported the oxidation of methane to organic oxygenates at 160 °C (pressure of methane 3.5 MPa) using metallic palladium dissolved in oleum. Methanol was obtained by the transformation of methane to methyl bisulfate and dimethyl sulphate and the subsequent hydrolysis of the ester. In a similar way, L. Chen et al., Energy and Fuels, 20 (2006) 915 reported the use of V₂O₅ in oleum at 180 °C (4.0 MPa pressure of methane). E.D. Park et al., Catal. Commun. 2 (2001) 187 and Appl. Catal. A 247 (2003) 269 reported, the selective oxidation of methane using *in situ* generated hydrogen peroxide and a Pd/C and Cu(CH₃COO)₂ catalyst system in the presence of trifluoroacetic acid (TFA) and trifluoroacetic anhydride (TFAA) as solvents. Pd/C is an *in situ* H₂O₂ generator, whereas Cu(CH₃COO)₂ is the oxidation catalyst. The reaction conditions were 80 °C, 5 mL solvent and total pressure of 47.64 atm (71.4% CH₄, 14.3% H₂, 14.3% O₂), Extreme care should be taken if one attempts to replicate experiments at these conditions as, at atmospheric pressure, H₂ concentrations above 4% in air have the potential to detonate. Additionally, the lower concentration limit for H₂ flammability and detonation in O₂ is expected to decrease with increasing pressure. In all cases, the hydrolysis of the ester that is formed under reaction conditions has to proceed in order to obtain the desired methanol product (i.e. methanol is not formed as a direct product from the reaction).

Kalvachev et al, Journal of Catalysis, vol. 186, no.1, p228-233, 1999 discloses vapor-phase selective oxidation of aliphatic hydrocarbons over gold deposited on mesoporas titanium silicates.

The inventors have recognized that an environmentally benign and economically attractive solution could be the use of O₂ and H₂O₂ as oxidants and the use of non-toxic solvents, such as water. Recently it has been shown by Qiang Yuan et al., Adv. Synth. Catal. 349 (2007) 1199, that it is possible to oxidize methane in an aqueous medium using metal chlorides and H₂O₂ wherein the catalytic system was based on the use of hydrogen peroxide in water using homogeneous transition metal chlorides (e.g. FeCl₃, COCl₂, RuCl₃, RhCl₃, PdCl₂, OsCl₃, IrCl₃, H₂PtCl₆, CUCl₂, and HAuCl₄). This method has several process disadvantages which include the homogeneous catalysts are highly soluble in water and therefore pose a separation problem from recycle and reuse. It would be highly preferable to use a heterogeneous catalyst that could be recovered and reused. In addition, these homogeneous catalysts show undesirable selectivities toward highly oxidized carbon species such as formic acid and CO₂. It is desired to have significantly higher selectivities for these oxidation reactions.

This invention, in one broad respect, is a process for forming an alcohol from a hydrocarbon, such as methanol from methane. The first process embodiment involves contacting hydrogen peroxide and a C₁-C₈ hydrocarbon in the presence of a heterogeneous catalyst in a liquid solution to convert the C₁-C₈ hydrocarbon to a corresponding C₁-C₈ alcohol, wherein the heterogeneous catalyst comprises gold on a solid support. By liquid solution it is meant any medium that is a liquid under the process conditions. The hydrocarbon can be fed, with or without other diluent, as either a gas or a liquid to the reaction, preferably at pressures from 1 atmospheres (atm), to 140 atm (101 - 14185 kPa)more preferably from 8 atm to 100 atm (810 - 10132 kPa), most preferably from 20 atm to 70 atm (2026 - 8106 kPa). That is, the process is conducted to maintain such pressures. Further as used herein a heterogeneous catalyst means one that is not being solubilized in the liquid solution. Typically the liquid solution is an aqueous media (e.g., distilled water). Importantly, the heterogeneous gold catalyst activates a hydrocarbon and H₂O₂ mixture to form a corresponding alcohol at temperatures preferably of from 0 °C to 200 °C, more preferably from 10°C to 100 °C, and most preferably from 30 °C to 90 °C. For example, it has been found that those heterogeneous gold catalysts can oxidize methane to methanol in water using hydrogen peroxide at temperatures as low as 30 °C thereby advantageously circumventing the need for high temperatures to activate methane where selectivity losses to CO or CO₂ are observed. Furthermore, it has been found that gold-based catalysts can be used to generate *in situ* H₂O₂ from H₂ and O₂ that is directly utilized to oxidize methane to methanol. Moreover, since these catalysts are not soluble in the liquid, they can be recovered and recycled for subsequent use in another reaction by standard separation techniques.

In a second process embodiment, the *in situ* generation of hydrogen peroxide at temperatures preferably from 0 °C to 200 °C, more preferably from 10 to 100 °C, and most preferably from 30 °C to 90 °C activates methane to form methanol over the gold comprising catalysts. The hydrocarbon is fed, with or without other diluent as either a gas or a liquid to the reaction, preferably at pressures from 1 atm to 140 atmospheres (atm), more preferably from 8 to 100 atm, and most preferably from 20 atm to 70 atm. It was found that adding palladium or copper to the gold comprising catalyst improved yields and selectivity to the desired alcohol.

In either process embodiment, this invention is a process for the production of an alcohol by contacting the hydrocarbon with a heterogeneous catalyst comprising gold. Other metals may be added to gold as a promoter to facilitate conversion of the hydrocarbon to desired products. Specifically, the addition of copper or palladium to the gold increased the alcohol selectivity of the catalyst. For example, a catalyst based on gold and palladium when contacting hydrogen peroxide and a C₁-C₈ hydrocarbon in the presence of the heterogeneous catalyst in a liquid solution converts the C₁-C₈ hydrocarbon to primarily the corresponding C₁-C₈ alcohol.

Likewise, in either process embodiment, the reaction temperature can be in the range from 30 °C to 90 °C; the process can be conducted under a total system pressure of from 1 atm to 140 atm; the hydrocarbon can be methane, ethane, propane, or a combination thereof (any combination of methane, ethane, and propane); the process can be conducted such that the hydrocarbon that is not in solution is at least partially in the gas phase at a pressure of up to 100 atm; the liquid solution can contain at least 90% water; the catalyst can contain gold and palladium; the catalyst can contain gold, palladium, and copper; the support can be composed of carbon, titania, ceria, iron oxide, copper oxide, silica, alumina, or a combination thereof; the catalyst can be prepared by impregnation, sol immobilization, chemical vapor infiltration, or a combination thereof; the catalyst can be calcined to increase the selectivity to the alcohol; the catalyst can contain gold in an amount of from 0.5 to 10 percent by weight based on the total weight of the catalyst; the catalyst can contain (a) gold in an amount of from 0.5 to 10 percent by weight based on the total weight of the catalyst, and (b) palladium, copper, or both, each in an amount of from 0 to 10 percent by weight based on the total weight of the catalyst; the catalyst can contain gold in an amount of from 2 to 10 percent by weight based on the total weight of the catalyst, and/or palladium and/or copper each in an amount up to 4 percent by weight based on the total weight of the catalyst; and any combination thereof.

This invention relates to processes and catalysts that convert C₁-C₃ hydrocarbons to a corresponding alcohol, such as methane to methanol, using a supported heterogeneous gold or promoted-gold catalyst. The hydrocarbons used in the practice of this invention generally contain from 1 to 8 carbon atoms (i.e., C₁-C₈). The hydrocarbons can be saturated or unsaturated, cyclic or linear or any combination thereof. In one embodiment, the hydrocarbon is methane. In another embodiment, the hydrocarbon is cyclohexane. In another embodiment, the hydrocarbon is octane. The hydrocarbons are selected such that in the practice of this invention a given hydrocarbon will react to form an alcohol. Mixtures of hydrocarbons can be used as the feedstock in the practice of this invention.

In the first embodiment of this process, hydrogen peroxide is used directly, without formation *in situ.* In this regard, hydrogen peroxide can be used as an aqueous mixture, as is commonly available commercially. The hydrogen peroxide can be employed in concentrated form, or can be diluted with additional water or other suitable solvent such as methanol. The amount of hydrogen peroxide used is effective to at least partially oxidize the hydrocarbon to its corresponding alcohol. Typically the amount of hydrogen peroxide used will be sufficient to maximize the amount of hydrocarbon being oxidized to its corresponding alcohol, without over-oxidation of the resulting product.

In this embodiment, the hydrocarbon can be feed, with or without other diluent, as either a gas or a liquid to the reaction medium containing the aqueous hydrogen peroxide solution, preferably at pressures from 1 atm to 140atm, more preferably from 8 atm to 100 atm, most preferably from 20 atm to 70 atm. Importantly, the heterogeneous gold catalyst activates a hydrocarbon and H₂O₂ mixture to form a corresponding alcohol at temperatures preferably from 0 °C to 200 °C, more preferably from 10 °C to 100 °C, and most preferably from 30°C to 90 °C.

While aqueous solutions of hydrogen peroxide can be used directly, the second embodiment of this process generates hydrogen peroxide in the aqueous media *in situ* from hydrogen and a source of oxygen. Any source of hydrogen can be used in the process of this invention such as is available commercially as well as, for example, molecular hydrogen obtained from the dehydrogenation of hydrocarbons and alcohols. Likewise, any source of oxygen can be employed, including air or pure oxygen.

When hydrogen peroxide is generated *in situ,* any amounts of hydrogen and oxygen can be employed in the process provided that the amount is sufficient to produce hydrogen peroxide in the desired quantities to achieve the desired conversion of hydrocarbon to a corresponding alcohol. The hydrogen peroxide is generated through the use of a heterogeneous catalyst in the liquid phase. By *in situ* it is meant that the hydrogen peroxide is produced within the reactor simultaneous (contemporaneously) with the oxidation of the hydrocarbon. For the application of *in situ* peroxide production with subsequent hydrocarbon oxidation, an aqueous media is placed in suitable reactor, wherein the aqueous media includes the catalyst. When a closed reactor is used, the hydrogen/oxygen mixture is typically mixed with methane and an optional diluent and pressurized up to a total pressure preferably from 1 to 140 atmospheres (atm), more preferably from 8 to 100 atm, most preferably from 20 to 70 atm. Preferably a ratio of H₂:O₂ from 1:5 to 5:1 with optional diluent are useful from forming the *in situ* hydrogen peroxide, more preferably ratios of H₂:O₂ from 1:3 to 3:1 are useful, and most preferably H₂:O₂ ratios of 1:2 to 2:1 are useful. It is advisable to employ H₂:O₂ ratios with appropriate hydrocarbon and diluent pressure to avoid using explosive mixtures.

The particular pressure for H₂, O₂, gaseous hydrocarbon and diluent used in a given reaction can vary depending on for example the equipment, the phase of hydrocarbon reactant , the hydrogen/oxygen ratio, and concentration of hydrocarbon either in the gas phase or present in any diluent (e.g. in an aqueous media). When the reactants, aqueous media, and catalyst are within the sealed reaction chamber, a reaction temperature is maintained preferably from 0 °C to 200 °C, more preferably from 10 °C to 100 °C, and most preferably from 30 °C to 90 °C.

The catalysts used in any process embodiment herein are heterogeneous gold-containing catalysts. The catalysts can contain other metals such as copper and palladium, for example, to facilitate *in situ* production of hydrogen peroxide and/or alkane oxidation. In one embodiment, the catalyst contains gold and palladium. In a second embodiment, the catalyst contains gold and copper. In another embodiment, the catalyst contains gold, palladium, and copper. The gold can be supported on a variety of materials, including but not limited to carbon, ceria, iron oxide, copper oxide, silica, titania, and alumina supports. It is further embodied that copper oxide or hydroxide may be used as a support for gold or palladium. The catalysts may be formed into a variety of shapes and sizes and by a variety of methods. For example, the support may be combined with a binder into extrudate or pellets for added strength and durability.

The catalysts can be made using a variety of well known methods. In one technique, a solution is used to load a metal onto a solid support. For example an aqueous gold solution is formed from a suitable salt such as HAuCl₄. If desired another metal may be added to solution. For example, palladium may be added such as through use of palladium chloride (PdCl₂). The aqueous solution's temperature, concentration, pH, and other variables can be adjusted depending on the desired characteristics of the final catalyst. The concentration of gold and other metal or metals can be adjusted relative to each other and relative to the amount of solid support being used in order to produce a final catalyst with the desired metal loading and relative composition on the support. In one embodiment, the support is slowly added to the aqueous metal solution, with stirring, to form a suspension where the metals are incorporated on the support. The catalyst may thereafter be dried and/or calcined, if desired, at a temperature of from 100 to 600 °C, though the temperature may vary depending on the composition of the catalyst. Alternatively, the catalyst can be formed by a variety of other well known techniques, such as impregnation to incipient wetness, deposition precipitation (with or without urea), sol immobilization, and chemical vapor deposition as shown in the examples.

The catalysts generally contain gold in amounts from preferably 0.001 to 10 percent by weight based on the total weight of the catalyst, more preferably in the range of 1 to 5 percent by weight, and most preferably in the range of 2 to 3 percent. Likewise, the catalysts generally contain palladium in amounts from preferably 0.001 to 10 percent by weight based on the total weight of the catalyst, more preferably in the range of 1 to 5 percent by weight, and most preferably in the range of 2 to 3 percent. In certain embodiments, the catalyst can include copper in amounts of from preferably 0.001 to 10 percent by weight, more preferably in the range of 1 to 5 percent and most preferably in the range of 2 to 3 percent. If other metals such as promoters are present, they are typically in an amount of from 0.001 to 5 percent by weight of the total catalyst.

The amount of catalyst employed in a given reaction can vary widely. The catalyst can be used in any amount that provides conversion (oxidation) of at least a portion of the hydrocarbon to be converted into a corresponding alcohol. It is possible to employ two or more catalysts in the practice of this invention, which might achieve a specific result that is unachievable with a single catalyst.

If the catalyst loses activity over time, standard regeneration techniques can be used to reactivate the catalyst, such as by burning off build up on the catalyst or treating the catalyst with fresh hydrogen peroxide solutions. Alternatively, fresh catalyst can be introduced.

The following examples are illustrative of this invention and are not intended to limit the scope of the invention or claims hereto. Unless otherwise denoted all percentages are by weight. Methane of 99.999% purity, 25% oxygen/carbon dioxide of 99.99% purity and 5% hydrogen/carbon dioxide obtained from BOC, were used without further purification. HAuCl₄·3H₂O, PdCl₂, CuCl₂ (99.99% purity) and activated carbon (G60, Aldrich) were supplied by Johnson Matthey and Sigma Aldrich. Titania (P25, 99.5%) was supplied by Degussa, ceria and alumina were supplied by Aldrich. All reactive compositions in this work have been analyzed as using the following procedures: The gas mixture of the reactor was removed using a gas sampling bag and analysis was performed using gas chromatography. The liquid-phase products were analyzed by High performance liquid chromatography (HPLC), ¹H nuclear magnetic resonance (NMR). D₂O was used as the lock reference. In the case of ¹H NMR analysis, a sealed capillary tube was prepared with a solution of TMS (tetramethylsilane) and CHCl₃ (chloroform). H₂O₂ yield was determined by titration of aliquots of the final filtered solution with acidified Ce(SO₄)₂ solutions were standardized against (NH₄)₂Fe(SO₄)₂·6H₂O using ferroin as indicator.

### Example 1: Preparation of a Au/TiO₂ catalyst by impregnation

The Au/TiO₂ catalyst is prepared by impregnation of an aqueous solution HAuCl₄·3H₂O onto the necessary amount of TiO₂ (Degussa, P25) support to achieve a final loading of 2.5% by weight. The gold solution is prepared by adding 5 grams of HAuCl₄-3H₂O to 250 mL deionized water with vigorous stirring. After the complete dissolution of the gold salt, 1.9 grams of the support is added very slowly into 5 ml of the solution and continuously stirred until it became homogeneous. The slurry is kept in the oven for 16 hrs at 110 °C. This catalyst is designated herein as catalyst E1.

### Example 2: Liquid phase oxidation of methane with hydrogen peroxide using a Au/TiO₂ catalyst

The catalytic oxidation of methane is carried out using a stainless-steel autoclave (Parr reactor) containing a Teflon liner vessel with total volume of 50 ml. A measured amount of the catalyst from Example 1 E1 corresponding to 10⁻⁵ mol of metal is added into the Teflon vessel, which was pre-charged with a 10 ml solution of distilled water and the desired amount of H₂O₂ (50 wt% H₂O₂, 0.005 mol of H₂O₂). The total volume of the reaction solution is 10 ml. The system is pressurized with methane to a fixed pressure (440 psi, 0.05 mol) after air in the reactor is removed with the reactant (purging 3 times with methane at 200 psi, 13.61 bar). The autoclave is heated to 90 °C. Once the reaction temperature is attained the solution is vigorously stirred at 1500 rpm and maintained at the reaction temperature for 2 hours. At the end of the reaction the autoclave is cooled with ice to a temperature of 12 °C to minimize the methanol volatility and loss. Products are subsequently analyzed and the results are shown in Table 1.

### Example 2.1: Liquid phase oxidation of methane with hydrogen peroxide using a calcined Au/Ti02 catalyst.

The oxidation process of Example 2 is repeated with the following modifications. Fresh catalyst E1 is calcined at 400 °C for 3 hours in static air before use in the reaction. Once the reaction temperature is attained the solution is vigorously stirred at 1500 rpm and maintained at the reaction temperature for 4 hours. Products are subsequently analyzed and the results are shown in Table 1. An improved methanol selectivity is observed.

### Example 3: Preparation of a AuPd/TiO₂ catalyst by impregnation

The AuPd/TiO₂ catalyst is prepared by impregnation of aqueous solutions of PdCl₂ and HAuCl₄·3H₂O onto the necessary amount of TiO₂ (Degussa, P25) support to achieve a final loading of 5% wt (2.5wt%Au-2.5wt%Pd) by weight. The gold solution is prepared by adding 5 grams of HAuCl₄·3H₂O to 250 mL deionized water. The Pd precursor (0.083 grams) is dissolved into 5 ml of the gold solution with vigorous stirring. After the complete dissolution of the salts, 1.9 grams of the support is added very slowly into the 5 ml of the solution and continuously stirred until it became homogeneous. The slurry is kept in the oven for 16 hrs at 110 °C. This catalyst is designated herein as catalyst E3.

### Example 4: Liquid phase oxidation of methane with hydrogen peroxide using a AuPd/TiO₂ catalyst

The oxidation process of Example 2 is carried out using the catalyst E3 of Example 3. Products are subsequently analyzed and the results are shown in Table 1.

### Example 5: Preparation of a AuPdCu/TiO₂ catalyst by impregnation

The AuPdCu/TiO₂ catalyst is prepared by impregnation of aqueous solutions of PdCl₂, CuCl₂ and HAuCl₄3H₂O onto the necessary amount of TiO₂ (Degussa, P25) support to achieve a final loading of 7.5% wt (2.5wt%Au-2.5wt%Pd-2.5wt%Cu) by weight. The gold solution is prepared by adding 5 grams of HAuCl₄·3H₂O to 250 mL deionized water. The Pd precursor (0.083 grams) is dissolved into 5 ml of the gold solution with vigorous stirring. After the complete dissolution of the salts, 1.9 grams of the the support is added very slowly into the 5 ml of the solution and continuously stirred until it became homogeneous. The slurry is kept in the oven for 16 hrs at 110 °C. This catalyst is designated herein as catalyst E5.

### Example 6: Liquid phase oxidation of methane with hydrogen peroxide using a AuPdCu/TiO₂ catalyst

The oxidation process of Example 2 is carried out using the catalyst E5 of Example 5 with the following modifications. Fresh catalyst E5 is calcined at 400 °C for 3 hours in static air before use in the reaction. The reaction temperature used is 50 °C. Once the reaction temperature is attained the solution is vigorously stirred at 1500 rpm and maintained at the reaction temperature for 0.5 hours. Products are subsequently analyzed and the results are shown in Table 1.

### Example 7: Liquid phase oxidation of methane with hydrogen peroxide using a calcined AuPd/TiO₂ catalyst

The oxidation process of Example 2 is repeated with the following modifications. Fresh catalyst E4 is calcined at 400 °C for 3 hours in static air before use in the reaction. Once the reaction temperature is attained the solution is vigorously stirred at 1500 rpm and maintained at the reaction temperature for 0.5 hours. Products are subsequently analyzed and the results are shown in Table 1.

### Example 7.1: Liquid phase oxidation of methane with hydrogen peroxide using a calcined AuPd/TiO₂ catalyst at lower temperatures

The oxidation process of Example 2 is repeated with the following modifications. Fresh catalyst E4 is used, and the reaction temperature is 50 °C. Once the reaction temperature is attained the solution is vigorously stirred at 1500 rpm and maintained at the reaction temperature for 0.5 hours. The catalyst is still quite active despite the very low temperature. Products are subsequently analyzed and the results are shown in Table 1.

### Example 8: Liquid phase oxidation of methane with hydrogen peroxide at higher pressures

The oxidation process of Example 2 is repeated with the following modifications. Fresh catalyst E4 is used, and is calcined at 400 °C for 3 hours in static air before use in the reaction. The reaction temperature used is 50 °C, while the methane pressure is increased to 50 atmospheres. Once the reaction temperature is attained the solution is vigorously stirred at 1500 rpm and maintained at the reaction temperature for 0.5 hours. Products are subsequently analyzed and the results are shown in Table 1. It is evident that more alcohol product is formed at higher methane pressures.

### Example 9: Preparation of a Au/C catalyst by impregnation

A 2.5 wt. % Au/C catalyst is prepared by the method of Example 1 where the TiO₂ support is substituted for carbon (Aldrich, G60). The dried Au/C catalyst is calcined at 400 °C for 3 hours in static air before use in the reaction. This catalyst is designated herein as catalyst E9. .Products are subsequently analyzed and the results are shown in Table 1.

### Example 10: Liquid phase oxidation of methane with hydrogen peroxide using a Au/C catalyst

The oxidation process of Example 2 is repeated with the following modifications. Fresh catalyst of E9 is used at a final reaction temperature of 50 °C. Once the reaction temperature is attained the solution is vigorously stirred at 1500 rpm and maintained at the reaction temperature for 4 hours. Products are subsequently analyzed and the results are shown in Table 1.

**Table 1. Catalytic data for the liquid phase oxidation of methane with hydrogen peroxide^{[a]}**

| Example | T (°C) | CH₄ pressure (atm) | Time (h) | Product amount (µmol) | | | CH₃OH Sel.% |
|---|---|---|---|---|---|---|---|
| | | | | CH₃OH ^{[b]} | HCOOH ^{[b]} | CO₂ in gas^{[c]} | |
| E2 | 90 | 30.9 | 2 | 1.86 | 1.86 | 69.44 | 2.5 |
| E2.1 | 90 | 30.9 | 4 | 0.71 | ND | 7.86 | 8.3 |
| E4 | 90 | 30.9 | 2 | 1.43 | - | 32.83 | 4.2 |
| E6 | 50 | 30.9 | 0.5 | 0.23 | ND | Trace | ND |
| E7 | 90 | 30.9 | 0.5 | 1.29 | ND | 4.49 | 22.3 |
| E7.1 | 50 | 30.9 | 0.5 | 0.57 | ND | 2.18 | 20.7 |
| E8 | 50 | 60 | 0.5 | 2.51 | ND | 0.36 | 87.5 |
| E10 | 90 | 30.9 | 4 | 0.43 | ND | 74.52 | 0.57 |

| | | | | | | | |
|---|---|---|---|---|---|---|---|
| ^{[a]}Reaction conditions: H₂O₂, 0.005 mol ; H₂O solvent, 10 mL, Catalyst (1.0 x 10⁻⁵ mol) unless otherwise. ^{[b]}Analysis using ¹H-NMR. ^{[c]}Analysis using Gas Chromatography. ND: Not detected | | | | | | | |

### Example 11: Liquid phase oxidation of methane with gas-phase H₂, O₂, and diluent using a AuPd/TiO₂ catalyst.

The catalytic oxidation of methane is carried out using a stainless-steel autoclave (Parr reactor) containing a Teflon vessel with total volume of 50 ml. Fresh catalyst E4 is calcined at 400 °C for 3 hours in static air, and a measured amount corresponding to 10⁻⁵ mol of metal is added into the Teflon vessel, which is pre-charged with a 10 ml solution of distilled water. The total volume of the reaction solution is 10 ml. The autoclave is purged three times with 5%/H₂/N₂ (7.8 atm) and then filled successively with 5%H₂/N₂ (5.4 atm), 25%O₂/N₂ (2.2 atm) and CH₄ (24 atm). The final molar ratio of hydrogen to oxygen is 1:1.7 and the total pressure is 31.6 atm. The autoclave is heated to 90 °C. After reaching the reaction temperature, the solution is vigorously stirred at 1500 rpm and maintained at the reaction temperature for 30 minutes. After reaction, the autoclave reactor is cooled with ice to below 15 °C (12 °C) to minimize the volatility and the loss of methanol. Products are subsequently analyzed and the results are shown in Table 2.

### Example 12: Liquid phase oxidation of methane with gas-phase H₂, O₂, and diluent using a AuPd/TiO₂ catalyst at lower temperatures

The oxidation process of Example 11 is repeated with the following modifications. The reaction temperature was 50 °C. Fresh catalyst E4 that is calcined at 400 °C for 3 hours in static air is used. Products are subsequently analyzed and the results are shown in Table 2.

### Example 13: Liquid phase oxidation of methane with gas-phase H₂, O₂, and diluent using a AuPd/TiO₂ and lower temperature

The oxidation process of Example 11 is repeated with the following modifications. Fresh catalyst E4 is calcined at 400 °C for 3 hours in static air and used, but with a reaction temperature of 30 °C. Instead of N₂, CO₂ is added as the diluent. The autoclave is purged three times with 5%/H₂/CO₂ (7.8 atm) and then filled successively with 5%H₂/CO₂ (21.8 atm), 25%O₂/CO₂ (8.7 atm) and CH₄ (24 am). The total pressure is 55.5 atm. The total reaction time is altered to be 4 hours. Gas phase CO₂ after reaction could not be measured for experiments with CO₂ as diluent. Products are subsequently analyzed and the results are shown in Table 2.

### Example 13.1: Liquid phase oxidation of methane with gas-phase O₂, and diluent using a AuPd/TiO₂.

The oxidation process of Example 11 was repeated with the following modifications. Fresh catalyst E4 that is calcined at 400 °C for 3 hours in static air and used at a reaction temperature of 50 °C. For this experiment, no H2 is used. The autoclave is purged three times with 25%O₂/CO₂ (7.8 atm) and then filled successively with 25%O₂/CO₂ (9.0 atm) and CH₄ (24 atm). The total pressure is 36 atm. The total reaction time is altered to be 4 hours. Gas phase CO₂ after reaction could not be measured for experiments with CO₂ as diluent. No products are detected, demonstrating the necessity for H₂ to form the active hydrogen peroxide in solution. Products are subsequently analyzed and the results are shown in Table 2.

### Example 14: Preparation of a AuPd/SiO₂ catalyst by impregnation.

The preparation method of E3 is followed using SiO₂ (99.8%, Degussa) instead of TiO₂ as the support material. The final result is a 2.5wt.%Au-2.5wt.%Pd/SiO₂ catalyst This material is calcined at 400 °C for 3 hours in static air before use in the reaction. This catalyst is designated herein as catalyst E14.

### Example 15: Preparation of a AuPd/CeO₂ catalyst by impregnation.

The preparation method of E3 is followed using CeO₂ (99.9%, Aldrich) instead of TiO₂ as the support material. The final result is a 2.5wt.%Au-2.5wt.%Pd/CeO₂ catalyst. This material is calcined at 400 °C for 3 hours in static air before use in the reaction. This catalyst is designated herein as catalyst E15.

### Example 16: Preparation of a AuPd/A1₂O₃ catalyst by impregnation.

The preparation method of E3 was followed using γ-Al₂O₃ (99.7%, Aldrich) instead of TiO₂ as the support material. The final result is a 2.5wt.%Au-2.5wt.%Pd/Al₂O₃ catalyst. This material is calcined at 400 °C for 3 hours in static air before use in the reaction. This catalyst is designated herein as catalyst E16.

### Example 17: Preparation of a AuPd/C catalyst by impregnation.

The preparation method of E3 is followed using C (G60, Aldrich) instead of TiO₂ as the support material. The final result is a 2.5wt.%Au-2.5wt.%Pd/C catalyst. This material is calcined at 400 °C for 3 hours in static air before use in the reaction. This catalyst is designated herein as catalyst E17.

### Example 18: Liquid phase oxidation of methane with gas-phase H₂, O₂, and diluent using a AuPd/SiO₂ catalyst.

The oxidation process of Example 11 is repeated with the following modifications. The reaction temperature is 50 °C. Fresh catalyst E14 that is calcined at 400 °C for 3 hours in static air is used. Products are subsequently analyzed and the results are shown in Table 2.

### Example 19: Liquid phase oxidation of methane with gas-phase H₂, O₂, and diluent using a AuPd/CeO₂ catalyst.

The oxidation process of Example 11 is repeated with the following modifications. The reaction temperature is 50 °C. Fresh catalyst E15 that is calcined at 400 °C for 3 hours in static air is used. Products are subsequently analyzed and the results are shown in Table 2.

### Example 20: Liquid phase oxidation of methane with gas-phase H₂, O₂, and diluent using a AuPd/y-Al_{z}O₃ catalyst.

The oxidation process of Example 11 is repeated with the following modifications. The reaction temperature is 50 °C. Fresh catalyst E16 that is calcined at 400 °C for 3 hours in static air is used. Products are subsequently analyzed and the results are shown in Table 2.

### Example 21: Liquid phase oxidation of methane with gas-phase H₂, O₂, and diluent using a AuPd/C catalyst.

The oxidation process of Example 11 is repeated with the following modifications. The reaction temperature is 50 °C. Fresh catalyst E17 that is calcined at 400 °C for 3 hours in static air is used. Products are subsequently analyzed and the results are shown in Table 2.

### Example 22: Preparation of a AuPdCu/TiO₂ catalyst by chemical vapor infiltration (CVI) and impregnation

Fresh dried catalyst from example E3 (1 gram) is placed into a vacuum finger flask containing copper acetylacetonate (0.103 grams, Aldrich) and a stirrer bar under reduced pressure within the range of 10⁻³ mbar. Under vacuum, the volatile copper precursor is deposited on the AuPd/TiO₂ catalyst to a nominal metal loading of 2.5wt% Cu. The final catalyst has a composition of 2.5%Au-2.5%Pd-2.5%Cu/TiO₂. Approximately 0.9 g of catalyst is recovered. This catalyst is designated herein as catalyst 22..

### Example 23: Liquid phase oxidation of methane with gas-phase H₂, O₂, and diluent using an impregnated AuPdCu/TiO₂ catalyst.

The oxidation process of Example 11 is repeated with the following modifications. The reaction temperature is 50 °C. Fresh catalyst E5 that is calcined at 400 °C for 3 hours in static air is used. A 27.6 mg catalyst charge is used in the reactor. Products are subsequently analyzed and the results are shown in Table 2.

### Example 23.1: Liquid phase oxidation of methane with gas-phase H₂, O₂, and diluent using an impregnated+CVI AuPdCuITiO₂ catalyst.

The oxidation process of Example 11 is repeated with the following modifications. The reaction temperature is 50 °C. Fresh catalyst E22 that is calcined at 400 °C for 3 hours in static air is used. A 10 mg catalyst charge is used in the reactor. Products are subsequently analyzed and the results are shown in Table 2. The result clearly show that the addition of Cu via CVI has produced a more active and selective catalyst as compared to Cu deposited by impregnation.

### Example 24: Preparation of a AuPd/TiO₂ catalyst by sol immobilization

An Au-Pd bimetallic sol (1:1 molar ratio) is prepared using aqueous PdCl₂ and HAuCl₄ solutions of 1.648 10⁻⁴ M. The desired amount of a polyvinyl alcohol (PVA), 1 wt % solution is added (PVA/Au (wt/wt) = 1.2) to form a dark-brown sol. After 30 minutes of sol generation, the colloid is immobilized by adding TiO₂ (Degussa P25). The solution is acidified to pH 1 by the addition of sulphuric acid under vigorous stirring. The amount of TiO₂ (2 grams) added is calculated as having a total final metal loading of 1% wt. After 2 hours the slurry is filtered, the catalyst washed thoroughly with distilled water and dried in air at 120 °C overnight. This catalyst is designated herein as catalyst E24.

### Example 25: Liquid phase oxidation of methane with gas-phase H₂, O₂, and diluent using a AuPd/TiO₂ catalyst prepared by sol immobilization.

The oxidation process of Example 11 is repeated with the following modifications. The reaction temperature is 50 °C. Fresh catalyst E24, 10 mg, is used without further treatment. Products are subsequently analyzed and the results are shown in Table 2.

**Table 2. Catalytic data for the liquid phase oxidation of methane with H₂ and O₂^{[a]}**

| Example | Partial Pressures (atm) | | | | Time (hr) | Temp (°C) | Product amount (µmol) | | | H₂O₂ remaining after reaction (µmol) |
|---|---|---|---|---|---|---|---|---|---|---|
| | O₂ | H₂ | CH₄ | inert | | | CH₃OH ^{[b]} | HCOOH ^{[b]} | CO₂ in gas | |
| E11 | 0.54 | 0.32 | 24 | 7.7 | 0.5 | 90 | 0.74 | 0 | 0.56 | 25 |
| E12 | 0.54 | 0.32 | 24 | 7.7 | 0.5 | 50 | 0.63 | 0 | 0.13 | 56 |
| E13 | 2.2 | 1.1 | 24 | 28 | 4 | 30 | 0.29 | 0 | ND^{[c]} | 25 |
| E13.1 | 2.2 | 0 | 24 | 7.5 | 4 | 50 | 0 | 0 | ND^{[c]} | ND |
| E18 | 0.54 | 0.32 | 24 | 7.7 | 0.5 | 50 | 0.30 | 0 | 1.31 | 58 |
| E19 | 0.54 | 0.32 | 24 | 7.7 | 0.5 | 50 | 3.9 | 0 | 0.94 | 47 |
| E20 | 0.54 | 0.32 | 24 | 7.7 | 0.5 | 50 | 0.24 | 0 | 0.27 | 36 |
| E21 | 0.54 | 0.32 | 24 | 7.7 | 0.5 | 50 | 1.83 | 0 | 0.40 | 36 |
| E23 | 0.54 | 0.32 | 24 | 7.7 | 0.5 | 50 | 0.23 | 0 | trace | 18 |
| E23.1 | 0.54 | 0.32 | 24 | 7.7 | 0.5 | 50 | 2.23 | 0 | 0.34 | 53 |
| E25 | 0.54 | 0.32 | 24 | 7.7 | 0.5 | 50 | 0.63 | 0 | 0.30 | 37 |

| | | | | | | | | | | |
|---|---|---|---|---|---|---|---|---|---|---|
| ^{[a]} Reaction conditions: H₂O solvent, 10 mL, Catalyst weight 1.0 x 10⁻⁵ mol metal unless otherwise noted ^{[b]}Analysis using ¹H-NMR. ^{[c]}Analysis using Gas Chromatography not determined because of the presence of CO₂ as gas diluent and reactive media. ND.= not determined. | | | | | | | | | | |

## Claims

1. A process for the production of an alcohol, comprising: contacting hydrogen peroxide and a C₁-C₈ hydrocarbon in the presence of a heterogeneous catalyst in a liquid solution to convert the C₁-C₈ hydrocarbon to a corresponding C₁-C₈ alcohol, wherein the heterogeneous catalyst comprises gold on a solid support, wherein the support is composed of carbon, titania, ceria, iron oxide, copper oxide, silica, alumina, or a combination thereof..

2. The process of claim 1, where the temperature is in the range from 30 °C to 90 °C.

3. The process of claim 1, where the process is conducted under a total system pressure of from 1 atm to 140 atmospheres.

4. The process of claim 1, where hydrocarbon is methane, ethane, propane, or a combination thereof.

5. The process of claim 1, wherein the process is conducted such that the hydrocarbon that is not in solution is at least partially in the gas phase at a pressure of up to 100 atmospheres.

6. The process of claim 1, wherein the liquid solution contains at least 90% water.

7. The process of claim 1, wherein the catalyst contains gold and palladium.

8. The process of claim 1, wherein the catalyst contains gold, palladium, and copper.

9. The process of claim 1, wherein the catalyst is prepared by impregnation, sol irrimobilization, chemical vapor infiltration, or a combination thereof.

10. The process of claim 1, wherein the catalyst has been calcined to increase the selectivity to the alcohol.

11. The process of claim 1, wherein the catalyst contains gold in an amount of from 0.5 to 10 percent by weight based on the total weight of the catalyst.

12. The process of claim 1, wherein the catalyst contains (a) gold in an amount of from 0.5 to 10 percent by weight based on the total weight of the catalyst, and (b) palladium, copper, or both, each in an amount of from 0 to 10 percent by weight based on the total weight of the catalyst.

13. The process of claim 1, wherein the catalyst contains gold in an amount of from 2 to 10 percent by weight based on the total weight of the catalyst, and/or palladium and/or copper each in an amount up to 4 percent by weight based on the total weight of the catalyst.

14. The process of claim 1, wherein the hydrogen peroxide is generated in situ by contacting H₂ and O₂ with an optional diluent in the presence of the heterogeneous catalyst to form hydrogen peroxide.

## Patentansprüche

1. Ein Verfahren zur Herstellung eines Alkohols, das Folgendes beinhaltet: In-Kontakt-Bringen von Wasserstoffperoxid und einem C₁-C₈-Kohlenwasserstoff in Gegenwart eines heterogenen Katalysators in einer flüssigen Lösung, um den C₁-C₈-Kohlenwasserstoff in einen entsprechenden C₁-C₈-Alkohol umzuwandeln, wobei der heterogene Katalysator Gold auf einem festen Träger beinhaltet, wobei der Träger aus Kohlenstoff, Titandioxid, Cerdioxid, Eisenoxid, Kupferoxid, Siliciumoxid, Aluminiumoxid oder einer Kombination daraus besteht.

2. Verfahren gemäß Anspruch 1, wobei die Temperatur in dem Bereich von 30 °C bis 90 °C liegt.

3. Verfahren gemäß Anspruch 1, wobei das Verfahren unter einem Gesamtsystemdruck von 1 atm bis 140 physikalische Atmosphären durchgeführt wird.

4. Verfahren gemäß Anspruch 1, wobei der Kohlenwasserstoff Methan, Ethan, Propan oder eine Kombination daraus ist.

5. Verfahren gemäß Anspruch 1, wobei das Verfahren so durchgeführt wird, dass sich der Kohlenwasserstoff, der sich nicht in der Lösung befindet, mindestens teilweise in der Gasphase bei einem Druck von bis zu 100 physikalischen Atmosphären befindet.

6. Verfahren gemäß Anspruch 1, wobei die flüssige Lösung mindestens 90 % Wasser enthält.

7. Verfahren gemäß Anspruch 1, wobei der Katalysator Gold und Palladium enthält.

8. Verfahren gemäß Anspruch 1, wobei der Katalysator Gold, Palladium und Kupfer enthält.

9. Verfahren gemäß Anspruch 1, wobei der Katalysator durch Imprägnierung, Sol-Immobilisierung, chemische Gasphaseninfiltration oder einer Kombination daraus zubereitet wird.

10. Verfahren gemäß Anspruch 1, wobei der Katalysator kalziniert worden ist, um die Selektivität hinsichtlich des Alkohols zu erhöhen.

11. Verfahren gemäß Anspruch 1, wobei der Katalysator Gold in einer Menge von 0,5 bis 10 Gewichtsprozent, bezogen auf das Gesamtgewicht des Katalysators, enthält.

12. Verfahren gemäß Anspruch 1, wobei der Katalysator (a) Gold in einer Menge von 0,5 bis 10 Gewichtsprozent, bezogen auf das Gesamtgewicht des Katalysators, und (b) Palladium, Kupfer oder beides, jeweils in einer Menge von 0 bis 10 Gewichtsprozent, bezogen auf das Gesamtgewicht des Katalysators, enthält.

13. Verfahren gemäß Anspruch 1, wobei der Katalysator Gold in einer Menge von 2 bis 10 Gewichtsprozent, bezogen auf das Gesamtgewicht des Katalysators, und/oder Palladium und/oder Kupfer, jeweils in einer Menge von bis zu 4 Gewichtsprozent, bezogen auf das Gesamtgewicht des Katalysators, enthält.

14. Verfahren gemäß Anspruch 1, wobei das Wasserstoffperoxid in situ durch das In-Kontakt-Bringen von H₂ und O₂ mit einem optionalen Verdünnungsmittel in Gegenwart des heterogenen Katalysators, um Wasserstoffperoxid zu bilden, erzeugt wird.

## Revendications

1. Un procédé pour la production d'un alcool, comprenant : la mise en contact de peroxyde d'hydrogène et d'un hydrocarbure en C₁ à C₈ en présence d'un catalyseur hétérogène dans une solution liquide pour convertir l'hydrocarbure en C₁ à C₈ en un alcool en C₁ à C₈ correspondant, dans lequel le catalyseur hétérogène comprend de l'or sur un support solide, le support étant composé de carbone, de dioxyde de titane, d'oxyde de cérium, d'oxyde de fer, d'oxyde de cuivre, de silice, d'alumine, ou d'une combinaison de ceux-ci.

2. Le procédé de la revendication 1, où la température est comprise dans la gamme allant de 30 °C à 90 °C.

3. Le procédé de la revendication 1, le procédé étant conduit sous une pression de système totale allant de 1 atm à 140 atmosphères.

4. Le procédé de la revendication 1, où l'hydrocarbure est le méthane, l'éthane, le propane, ou une combinaison de ceux-ci.

5. Le procédé de la revendication 1, le procédé étant conduit de sorte que l'hydrocarbure qui n'est pas en solution soit au moins partiellement dans la phase gazeuse à une pression allant jusqu'à 100 atmosphères.

6. Le procédé de la revendication 1, dans lequel la solution liquide contient au moins 90 % d'eau.

7. Le procédé de la revendication 1, dans lequel le catalyseur contient de l'or et du palladium.

8. Le procédé de la revendication 1, dans lequel le catalyseur contient de l'or, du palladium, et du cuivre.

9. Le procédé de la revendication 1, dans lequel le catalyseur est préparé par imprégnation, immobilisation du sol, infiltration en phase vapeur, ou une combinaison de celles-ci.

10. Le procédé de la revendication 1, dans lequel le catalyseur a été calciné pour augmenter la sélectivité à l'alcool.

11. Le procédé de la revendication 1, dans lequel le catalyseur contient de l'or dans une quantité allant de 0,5 à 10 pour cent en poids rapporté au poids total du catalyseur.

12. Le procédé de la revendication 1, dans lequel le catalyseur contient (a) de l'or dans une quantité allant de 0,5 à 10 pour cent en poids rapporté au poids total du catalyseur, et (b) du palladium, du cuivre, ou les deux, chacun dans une quantité allant de 0 à 10 pour cent en poids rapporté au poids total du catalyseur.

13. Le procédé de la revendication 1, dans lequel le catalyseur contient de l'or dans une quantité allant de 2 à 10 pour cent en poids rapporté au poids total du catalyseur, et/ou du palladium et/ou du cuivre, chacun dans une quantité allant jusqu'à 4 pour cent en poids rapporté au poids total du catalyseur.

14. Le procédé de la revendication 1, dans lequel le peroxyde d'hydrogène est généré in situ en mettant en contact H₂ et O₂ avec un diluant optionnel en présence du catalyseur hétérogène pour former du peroxyde d'hydrogène.
